# EUROPEAN PATENT SPECIFICATION

(11) **EP 2 123 328 B1**
(45) Date of publication and mention of the grant of the patent: **04.05.2011**
(21) Application number: 09472001.8
(22) Date of filing: 24.02.2009
(51) Int. Cl.: A61P 25/28, C07K 5/062, C07K 5/083, C07D 491/02, A61K 38/05, A61K 38/06, A61K 47/48

(54) **Galanthamine derivatives, methods for their obtaining and use**
Galanthamin-Derivate, Verfahren zu deren Herstellung und Verwendung
Dérivés de galanthamine, procédés pour leur obtention et utilisation

(30) Priority: 23.05.2008 BG 11014108
(43) Date of publication of application: 25.11.2009
(73) Proprietor: Sopharma AD, 1220 Sofia (BG)
(72) Inventor: Vezenkov, Lubomir Todorov, Sofia 1756 (BG); Georgieva, Mariya Georgieva, Sofia 1756 (BG); Danalev, Dancho Lubenov, Sofia 1700 (BG); Ivanov, Chavdar Borisov, Sofia 1784 (BG); Bakalova, Anastassia Todorova, Sofia 1309 (BG); Hristov, Krum Krumov, Sofia 1000 (BG); Mitev, Vanyo Ivanov, Sofia 1113 (BG); Checler, Frederic, 06410 Biot (FR); Sevalle, Jean, 06100 Nice (FR)

(56) References cited:
- EP-A- 1 576 955
- WO-A-01/74820
- WO-A-2008/022365
- US-A1- 2005 182 044
- US-A1- 2006 003 989
- GEORGIEVA M ET AL: "Design and synthesis of new .gamma.-secretase inhibitors" COMPTES RENDUS DE L'ACADEMIE BULGARE DES SCIENCES, SOFIA, GB, vol. 57, no. 9, 1 January 2004 (2004-01-01), pages 13-18, XP008106341 ISSN: 0861-1459

## Description

### FIELD OF THE INVENTION

This invention relates to new compounds that are galanthamine derivatives, to the methods of their obtaining and to said products for use in prophylaxis and/or treatment of neurodegenerative diseases, senile dementia and Alzheimer's disease.

### BACKGROUND OF THE INVENTION

Dementia is a syndrome characterized by decline of multiple cognitive functions sufficiently significant to disturb everyday activities of the patients. With dementia development, cognitive and behavioral disturbances lead to progressive loss of patient autonomy. Dementia is one of the most significant health and social problems of the 21^{st} century, together with vascular and oncological diseases. Morbidity increases significantly with aging and doubles every 5 years after the age of 60.

Alzheimer's disease (AD) is the cause for almost 50% of dementia cases in the third age, followed by vascular dementia causing 20% of all dementia cases, dementia with Levi bodies (10%), front-temporal (10%) and sub-cortex degenerative dementia (5%). The other forms represent totally 5% of all dementia types.

The most important biochemical changes observed in the brain of AD patients are decrease of acetylcholine neurotransmitter levels and of the related enzymes (cholintransferase and acetylcholinesterase) in the hippocampus and in the cortex part of the brain. The cholinergic system is important for learning and memory. Acetylcholinesterase plays an important role in AD development as this enzyme is responsible for the splitting of acetylcholine neurotransmitter and it also increases the aggregation of β-amyloid peptides that cause the formation of plates in the synaptic regions of cholinergic neurotransmission. A possible approach for AD treatment is to restore the acetylcholine level by inhibiting acetylcholinesterase using reversible inhibitors. Acetylcholinesterase inhibitor galanthamine / 4a*S*, 6*R*, 8a*S*)-4a,5,9,10,11,12-Hexahydro-3-methoxy-11-methyl-6H-benzofuro[3a,3,2-ef][2]benzazepin-6-ol/ is less toxic and absorbed better by tacrine and physostigmine. This plant alkaloid isolated from Amaryllidaceae is a centrally active, competitive and reversible acetylcholinesterase inhibitor and it has been proved that it causes a significant improve of cognitive manifestations in AD patients. In national medicine, an alcoholic extract of the overground part of this plant is used in various heart disorders, myocarditis, angina pectoris etc. Galanthamine hydrobromide is used for treatment of neuromuscular diseases, myasthenia gravis in particular, as well as in some neurological diseases as polyomyelitis. In addition, galanthamine is prescribed also in glaucoma, as an anticurare agent in anesthesia and for modifying the effects of muscarine receptor blocking drugs as scopolamine for example. Galanthamine acts as a weak parasympathicomimetic with indirect effect at the level of smooth muscles. Galanthamine manifests nicotine-cholinergic receptor activity, mono- and polysynaptic reflex activity, antagonistic effect on respiratory centre suppressing the activity of morphine and other narcotic analgesics. Pharmacokinetic behaviour of galanthamine differs in many aspects from the behaviour of its pharmacological analogues neostigmine and pyridostigmine, i.e.: it has lower toxicity, more rapid absorption, higher bioavailability, slower elimination.

Characteristic of AD is the precipitation of the hydrophobic β-amyloid peptide (Aβ) in the core of senile plaques and on the walls of cerebral blood vessels. Aβ manifests a neurotoxic and inflammatory effect in the tissues. It varies weakly in length and exists in two predominant forms: a shorter one, with 40 residues β(1-40) found mainly in the cerebrovascular amyloid, and a longer one, with 42 residues β(1-42), which is the main component of amyloid plaques. Aβ40,42 have been obtained during proteolytic processes of splitting of wide regions of 695-770 amino-acid residues β-amyloid precursor protein (βAPP), and once released they proceed to aggregation. Aβ obtaining from APP is performed by splittings catalyzed by various proteases known as secretases. APP splitting in the N-end at Aβ1 and Aβ11 is catalyzed by an aspartic acid protease called β-secretase. C-end splitting of Aβ at Aβ40 and Aβ42, which spreads in the transmembrane region of APP is due to the effect of proteases called γ-secretases. The inhibition of β- and γ-secretases presupposes a decrease in Aβ production and potential delay of AD progress.

Elan Pharmaceuticals Inc and Eli Liily and Company have created a class of low-molecular inhibitors of γ- secretase activity, which potentially reduce the production of Aβ peptide without significantly affecting the secretion of α and β forms of APP, *in vitro* and *in vivo* (WO 9822441 and WO 9822494). In the tests of compounds of this drug series, a dose-dependent reduction of Aβ-levels in the brain was observed with a peak 3 hours after administration, while the significant reduction of Aβ levels was maintained till the 18^{th} hours. The most active compound of this group is N-[N-(3,5-difluorophenylacetyl)-L-alanyl]-S-phenylglycin tertiary butyl ester (DAPT). The primary mechanism of action of DAPT is the functional inhibition of γ-secretase and not the inhibition of β- or α-secretase activity. No signs have been established of any cellular toxicity *in vitro* and *in vivo* during the tests.

Georgieva et al. (Comptes Rendus de l'Academie Bulgare des Sciences (2004) vol. 57, no. 9, pages 13-18) describe dipeptide derivatives as inhibitors of γ-secretase activity. WO 01/74820 describes dipeptide amide derivatives of galanthamine as possible condidates for use in the treatment of Alzheimer's disease.

Due to the multifactor character of neurodegenerative diseases, AD in particular, a combined therapy is advisable for their treatment. The technical problem posed to this invention is the creation of compounds that posses combined pharmacological properties, especially inhibition of acetylcholinesterase and butyrlcholinesterase activity and γ-secretase activity.

### DETAILED DESCRIPTION OF THE INVENTION

This invention relates to new compounds that are galanthamine derivatives, containing a peptide fragment, di- and tripeptides in particular of general formula I: 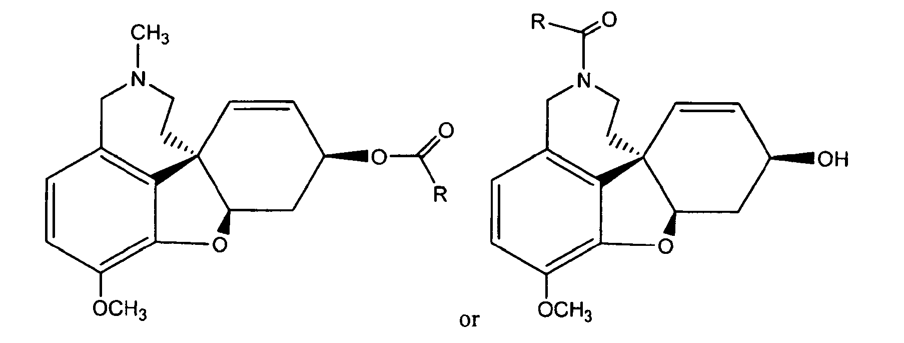 wherein:
R is a dipeptide or a tripeptide that contains in the side chain of aminoacids and/or at its N-end one or more alkyl, aryl, alkylaryl, phenyl, alkylphenyl, alkoxyphenyl residues, and particularly for side chains of aminoacids -CH(CH₃)₂, -CH₂C₆H₅, -CH₂CH(CH₃)₂; and for the N-end phenyl, alkylphenyl, alkoxyphenyl, halogenphenyl, wherein the halogen atoms being one or more Cl, F, Br;
as well as their diastereoisomers or their acid addition salts.

The invention includes also methods for obtaining of the new galanthamine derivatives. In one embodiment of the invention, galanthamine derivatives are obtained by condensing galanthamine or norgalanthamine with dipeptides or tripeptide free acids, substituted in certain cases, under the effect of a condensing agent and in the presence of a base in a medium of organic solvent.

In a subsequent embodiment of the invention, galanthamine derivatives are obtained by the interaction of Boc-Gly-OCH₂CN (tert-butyloxycarbonyl glycine cyanmethyl ester) with galanthamine in the presence of a condensing agent in a medium of minimum quantity of organic solvent; the obtained ester 6-O-(Boc-glycyl)-galanthamine is isolated by acidifying and subsequent removal of non-reacted products by extraction with organic solvent, alkalization of the water-acid phase and subsequent extraction of the target product using an organic solvent, followed by deblocking of Boc-protective group with an acid and treatment with diethyl ether; the obtained ester is condensed using different dipeptides or tripeptide free acids, substituted in certain cases, under the effect of a condensing agent in a medium of organic solvent and in the presence of a base at room temperature; the obtained raw galanthamine derivatives are subjected to recrystallization.

Dipeptides are synthesized in the form of free acids, substituted in certain cases, that are further included in reactions for production of galanthamine derivatives. With the purpose of obtaining dipeptide-free acids, N-(3,4-dichlorophenyl)-D,L-alanine is condensed with esters of aminoacids under the effect of a condensing agent in a medium of an organic solvent and in the presence of a base at room temperature; the produced esters are then subjected to alkaline hydrolysis in an alcohol medium at room temperature, the obtained compounds being purified and recrystallized. The required N-(3,4-dichlorophenyl)-D,L-alanine is obtained by means of alkylation of 3,4-dichloroaniline using 2-chloropropanic acid with yields and constants corresponding to those in literature (US 3 598 859); N-(3,4-dichlorophenyl)-D,L-alanine is further condensed with esters of amino acids under the effect of HBTU or TBTU or TCTU in a medium of an organic solvent selected from the group of CH₂Cl₂, DMF, CHCl₃, EtOAc and in the presence of a base selected from the group of Et₃N, Me₃N, pyridine, tributylamine, DIPEA, at room temperature; the obtained compounds are then subjected to hydrolysis with an excess of 2N NaOH in an alcoholic medium selected from the group of MeOH, EtOH, butanol, propanol, isopropanol, at room temperature and performance of reaction till ester depletion, which is monitored by chromatography, and further compounds are purified by processing with water solution of NaHCO₃ and subsequent recrystallization from EtOAc/diethyl ether/petroleum ether or diethyl/petroleum ether.

The dipeptide free acids obtained under this method are in the form of diastereomeric mixtures, which is obvious from the data from RP-HPLC and NMR analyses. In ¹H and ¹³C NMR-spectra and in RP-HPLC of dipeptides, all signals are doubled which is due to the presence of a mixture of R and S isomers.

Dipeptide production in the form of free acids, substituted in this case, the scheme below is followed:

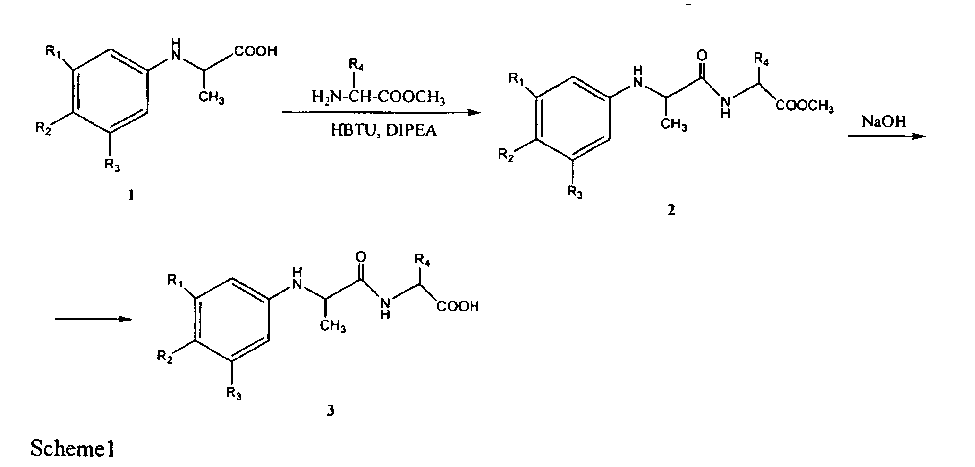

Wherein R₁, R₂, R₃ can be halogen, alkoxy, aryloxy, and the halogen atom can be Cl, F, Br; R₄ can be hydrogen, alkyl, aryl, alkylaryl, heteroalkyl, heteroaryl residues and in particular: - CH (CH₃)₂, -CH₂C₆H₅, -CH₂CH(CH₃)₂.

-The ester 6-O-(Boc-glycil)-galanthamine being synthesized from Boc-Gly-OCH₂CN and galanthamine in the presence of condensing agent base DBU in a medium of minimum quantity of organic solvent selected from the group of CH₂Cl₂, DMF, CHCl₃, EtOAc, dimethylsulfoxide; it is isolated as a pure product by acidifying with diluted HCl and extraction of non-reacted products with an organic solvent selected from the group of CH₂Cl₂, CHCl₃, EtOAc, alkalization of the water-acid phase to pH=8 and subsequent extraction of the target product using an organic solvent selected from the group of CH₂Cl₂, CHCl₃, EtOAc, and after deblocking of Boc-protective group with an acid selected from the group of TFA, hydrochloric acid/organic solvent, HBr/AcOH and subsequent treatment with diethyl ether, the ester is condensed using different dipeptide free acids, substituted in certain case, under the effect of HBTU or TBTU or TCTU in an organic solvent medium selected from the group of CH₂Cl₂, DMF, CHCl₃, EtOAc, dimethylsulphoxide in the presence of a base selected from the group of Et₃N, Me₃N, pyridine, tributylamine, DIPEA at room temperature, the obtained raw products produce crystalline products after re-crystallization. The scheme below is followed for the obtaining of galanthamine derivatives: 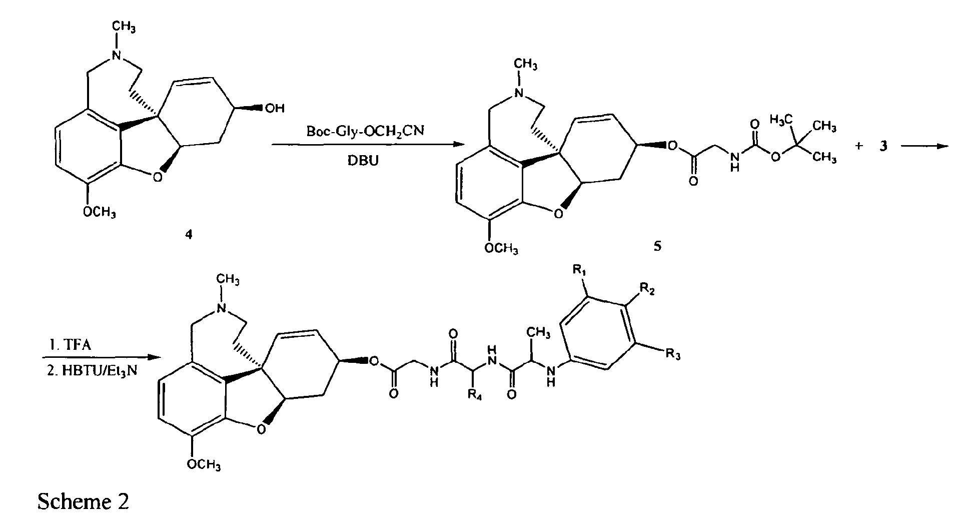 Wherein R₁, R₂, R₃ can be halogen, alkoxy, aryloxy, and the halogen atom can be Cl, F, Br; R₄ can be hydrogen, alkyl, aryl, alkylaryl, heteroalkyl, heteroaryl residues and in particular -CH(CH₃)₂, -CH₂C₆H₅, -CH₂CH(CH₃)₂.

In another embodiment of the invention galanthamine molecule is directly condensed with dipeptide or tripeptide free acids, substituted in certain cases, under the effect of a condensing agent selected from the group of diisopropylcarbodiimide, dicyclohexyl carbodiimide, N-cyclohexyl-N-[β-(N-methylmorpholino)-ethyl-] carbodiimide and in the presence of a base selected from the group of DMAP, Et₃N, Me₃N, pyridine, tributylamine, DIPEA in an organic solvent medium selected from the group of CH₂Cl₂, DMF, CHCl₃, EtOAc, dimethylsulfoxide at room temperature, where products are obtained having physicochemical characteristics identical with those of the galanthamine derivatives obtained under scheme 2, and in this embodiment of obtaining galanthamine derivatives, the scheme below is followed: 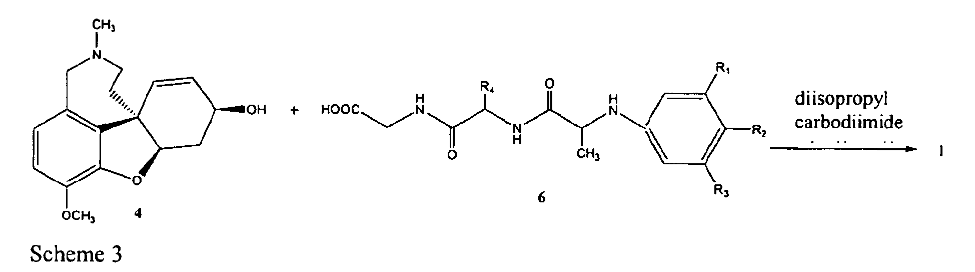 Wherein R₁, R₂, R₃ can be halogen, alkoxy, aryloxy, and the halogen atom can be Cl, F, Br; R₄ can be hydrogen, alkyl, aryl, alkylaryl, heteroalkyl, heteroaryl residues and in particular -CH(CH₃)₂, -CH₂C₆H₅, -CH₂CH(CH₃)₂.

The advantage of scheme 3 is that the reaction takes place quantitatively as it is monitored by chromatography. The compounds are in the form of diastereomeric mixtures according to the data from RP-HPLC and NMR analyses.

In the next embodiment of the invention, norgalanthamine is included in condensation with dipeptides or tripeptide free acids, substituted in certain cases, under the effect of a condensing agent selected from the group of diisopropyl carbodiimide, dicyclohexyl carbodiimide, N- dicyclohexyl-N-[β-(N-methylmorpholino)-ethyl-] carbodiimide and in the presence of a base selected from the group of DMAP, Et₃N, Me₃N, pyridine, tributylamine, DIPEA in an organic solvent medium selected from the group of CH₂Cl₂, DMF, CHCl₃, EtOAc, dimethylsulfoxide at room temperature, where obtained raw products produce crystalline products after recrystalization, and in this embodiment of obtaining galanthamine derivatives, the scheme below is followed: 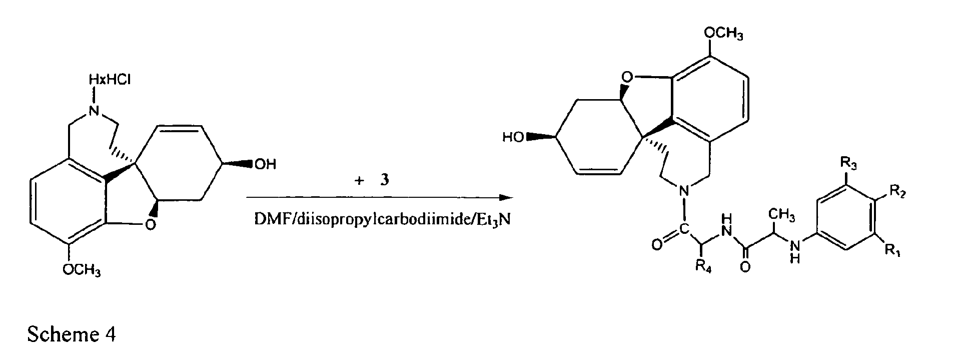 Wherein R₁, R₂, R₃ can be halogen, alcoxy, aryloxy, and the halogen atom can be Cl, F, Br; R₄ can be hydrogen, alkyl, aryl, alkylaryl, heteroalkyl, heteroaryl residues and in particular -CH(CH₃)₂, -CH₂C₆H₅, -CH₂CH(CH₃)₂.

The compounds are in the form of diastereomeric mixtures according to the data from HPLC and NMR analyses.

The compounds according to the invention manifest acetylcholinesterase- and butylrylcholinesterase-inhibiting activity and gamma-secretase inhibition activity.

The compounds according to the invention, as well as their diastereoisomers and acid addition salts can be used as active substances in pharmaceutical forms for example, for treatment and/or prophylaxis of neurodegenerative diseases, and particularly senile dementia and AD.

The invention relates also to pharmaceutical formulations that contain a therapeutically active quantity of at least one of the compounds obtained according to the invention and a pharmaceutically acceptable carrier and/or excipients. The pharmaceutical formulation can be intended for oral, inhalation, sublingual, parenteral, rectal, transdermal, intravenous administration.

The new galanthamine derivatives obtained according to the invention combine both effects: inhibition of acetylcholinesterase and butylcholinesterase activity due to the galanthamine molecule and inhibition of γ-secretase activity, inherent in the peptide fragment. The choice of such an approach has been determined by the fact that acetylcholinesterase enzyme induces the aggregation of Aβ on the one part, and on the other part, that Aβ increases the acetylcholinesterase level. It is assumed on the basis of these data that Aβ - acetylcholinesterase cycle enhances Aβ accumulation in the brain. Therefore, with the creation of these compounds a simultaneous effect is achieved on both pathological factors for the origination and development of the disease, to a much higher degree than the classical acetylcholinesterase inhibitors or the known γ-secretase inhibitors do.

Abbreviations used in the text:
For all amino acids their three-letter symbols are used according to the nomenclature approved by IUPAC.
Aβ - β - amyloid peptide
βAPP - β- amyloid protein precursor
Boc- tret-butyloxycarbonyl
CDCl₃- deuterated chloroform
DAPT - N-[N-(3,5-difluorophenylacetyl)-L-alanyl]-*S*-phenylglycin tertiary butyl ester DCC- N, N' - dicyclohexylcarbodiimidide
DCM - dichloromethan
DIPEA- diisopropylethylamine
DMAP- 4-N, N-(dimethylamino)-pyridine
DMF- N,N-dimethylformamide
DMSO- dimethylsulphoxide
DTNB- 5, 5'-ditiobis-2-nitrobenzoic acid
EtOH- ethanol
Et₃N- triethylamine
EtOAc- ethylacetate
HBTU- O-[(1-OH-benzotriazol)-1-yl]-1,1,3,3- tetramethylcarbamide hexafluorophosphate HOBt-1-hydroxybenzotriazol
HPLC- high-performance liquid chromatography
RP-HPLC - reverse phase high-performance liquid chromatography
IC₅₀- concentration providing 50% inhibition of enzyme activity
IR- Infrared spectroscopy
MeOH- methanol
MS- Mass spectrometry
NMR- Nuclear magnetic resonance
TBTU- *O*-(benzotriazol-1-yl)-1,1,3,3- tetramethylcarbamide tetrafluoroborate
TCTU- *O*-(1H-6-chlorobenzotriazol-1-yl)-1,1,3,3- tetramethylcarbamide tetrafluoroborate TFA- trifluoracetic acid
THF- tetrahydrofuran
TLC- Thin-layer chromatograpgy
TMS- tetramethylsilane

This invention is illustrated by the following synthetic and biological examples

### Examples:

### Comparative example 1. Synthesis of N-(3,4-dichlorophenyl)-D,L-alanine /according to US 3 598 859/

To a solution of 3,4-dichloroaniline (24.30 g 150.0000 mmol) in 2-propanol (76 ml) were added H₂O (4.5 ml) and 2-chloropropanic acid (25.73 ml 300.1900 mmol). The reaction mixture was heated to 40 ⁰C, then was added in portions NaHCO₃ (50.66 g 603.1000 mmol). The reaction mixture was boiled for 113 hours. The reaction mixture after cooling was poured in H₂O (900 ml). Then the non-reacted 3,4-dichloroaniline was removed by filtration. The filtrate was acidified to pH=3-4 with 2N HCl. After that the obtained precipitate was filtered, washed with H₂O and dried over KOH.

A crystal product is obtained with weight 20, 66 g and m.p. 142-144 ⁰C, yield 59,1 %, chromatographically pure in system EtOAc:hexane=4:1.
IR [cm⁻¹]: 3351, 2977, 1724, 1643, 1604, 1019, 717.

### Example 2. Synthesis of dipeptides - esters, derivatives of N-(3,4-dichlorophenyl)-D,L-alanine

To a solution of an ester of amino acid hydrochloride (12,5000 mmol) or hydrobromide in CH₂Cl₂ under cooling to 0⁰C were added 12,4500 mmol Et₃N, 1.2-fold excess of N-(3,4-dichlorophenyl)-alanine (15,0000 mmol), 1.21-fold excess of HBTU (15,1300 mmol) and 1.22-fold excess of DIPEA (15,2500 mmol). The reaction mixture was stirred at room temperature for several hours, then transfered in a separation funnel. The organic phase was washed in succession with 5% NaHCO₃, H₂O, 2N HCl and H₂O till a negative reaction for chlorine ions; dried with Na₂SO₄ and the solvent was distilled under vacuum.

Example 2a N-[N-(3,4-dichlorophenyl)-D,L-alanyl]-L-phenylalanine methyl esther The obtained raw product is oil that crystallizes when treated with diethyl ether. 3,86 g m. p. 120-123 °C, yield 78,1 %, chromatographically pure in systems CHCl₃:MeOH=9:1, n-BuOH:AcOH:H₂O= 3:1:1; EtOAc:hexane=1:1; acetone:MeOH=3: 1.
IR [cm⁻¹]: 3382, 3355, 3291, 3086, 3064, 2990, 2975, 2956, 2890, 1746, 1724, 1661, 1600, 1242.

### Example 2b N-[N-(3,4-dichlorophenyl)-D,L-alanyl]-L-valin methyl ester

The obtained product is oil with weight 4,05 g yield 93,1 %, chromatographically in systems n-BuOH:AcOH:H₂O= 3:1:1 and EtOAc:hexane=1:1.
IR [cm⁻¹]: 3367, 3294, 3073 2965, 2934, 2875,1732, 1649, 1600, 1382,1372, 1242.

### Example 2c N-[N-(3,4-dichlorophenyl)-D,L-alanyl]-L-leucine methyl ester

The obtained raw product is an oil that gives an amorphous product when treated with diethyl ether /petroleum ether with weight 1,06 g and m. p. 122-132 °C, yiled 73,0 %, chromatographically pure in systems n-BuOH:AcOH:H₂O= 3:1:1 and EtOAc:hexane=1:1. IR [cm⁻¹]: 3370, 3248, 3077, 2978, 2956, 2872, 1732, 1649, 1600, 1384, 1369, 1247.

### Example 2d N-[N-(3,4-dichlorophenyl)-D,L-alanyl]-glycine ethyl ester

The obtained raw product is oil that crystallizes when treated with diethyl ether. Weight 1,92 g m. p. 84-87 °C, yield 72,2 %, chromatographically pure in systems n-BuOH:AcOH:H₂O= 3:1:1 and EtOAc:hexane=1:1. [α]²²₅₄₆ +6 (cl MeOH).
IR [cm⁻¹]: 3372, 2996, 2928 1732, 1660, 1627, 1234.
¹H NMR (CDCl₃): δ (ppm)=12.0 (1H, OH), 7.22 (d, J=8.7 Hz, 1H, Ar-H), 7.05 (br.t, J=5.2 Hz, 1H, NH), 6.70 (d, J= 2.8 Hz, 1H, Ar-H), 6.46 (dd, J= 2.8, 8.7 Hz, 1H, Ar-H), 4.19 (q, J= 7.1 Hz, 2H, CH₂CH₃), 4.02 (dd, J=5.2, 17.0 Hz, NHCH₂ 3.82 (q, J=7.0 Hz, 1H, CHCH₃), 1.53 (d, J=7.0 Hz, 3H, CHCH₃), 1.26 (t, J=7.1 Hz, 3H, CH₂CH₃)
¹³C NMR (CDCl₃): 14.8 (CH₃), 20.2 (CH₃), 41.8 (CH₂), 55.5 (CH), 62.3 (CH₂CH₃), 113.9 (Ar), 115.7 (Ar), 122.6 (C-Cl), 131.5 (Ar), 133.7 (C-Cl), 146.6 (C-NH-CH), 170.2 (C=O), 174.4 (C=O).

### Example 3. Hydrolysis of dipeptides -esters to dipeptides-free acids

To a solution of the esters specified in Example 2 in MeOH was added an excess of 2N NaOH. The reaction mixture was stirred at room temperature and the depletion of starting components was controled using TLC in a system of EtOAc:hexane=1:1. The reaction was terminated upon depletion of the ester and MeOH after neutralization to pH=7 was removed under vacuum. The water-alkaline phase was acidified with 2N HCl to pH=2-3, then the target product was extracted using EtOAc. The product was extracted from the organic phase using 5% NaHCO₃, then the water-alkaline phase was acidified using 2N HCl to pH=2-3 and extracted again using EtOAc, washed with H₂O till a negative reaction for chlorine ions; dried with Na₂SO₄ and the solvent was distilled under vacuum.

### Example 3a N-[N-(3,4-dichlorophenyl)-D,L-alanyl]-L-phenylamine

The obtained product is amorphous. Weight 3,44 g yield 94,3 %, chromatographically pure in systems CHCl₃:MeOH=9:1, n-BuOH:AcOH:H₂O= 3:1:1; EtOAc:hexane=1:1; acetone:MeOH=3:1. [α]²²₅₄₆+18 (c1 MeOH).
IR [cm⁻¹]: 3375, 3370 3064, 3030, 3100-2500, 2978, 2933, 1724, 1648, 1600, 1240, 918.
¹H NMR (DMSO): δ (ppm)=12.7 (br.s, 1H, OH), 8.20 (d, J= 8.4 Hz, 1H, NH), 7.40-6.10 (9H, Ar-H), 4.44 (ddd, J=4.3, 8.7, 13.0 Hz, 1H, CHCH₂), 3.80 (q, J= 6.6 Hz, 1H, CHCH₃), 3.00 (m, 2H, CHCH₂), 1.22 (d, 3H, CHCH₃).
¹³C NMR (DMSO): 18.6 (CH₃), 36.8 (36.6) (CHCH₂), 52.1 (51.9) (CHCH₂), 53.1 (52.9) (CHCH₃), 112.6-138.0 (Ar), 147.8 (147.7) (CNHCH), 173.3 (173.2) (C=O), 172.8 (172.8) (C=O).

### Example 3b N-[N-(3,4-dichlorophenyl)-D,L-alanyl]-L-valine

The product obtained is amorphous. Weight 3,60 g yield 98,0 %, chromatographically pure in systems CHCl₃:MeOH=9:1, n-BuOH:AcOH:H₂O= 3:1:1; EtOAc:hexane=1:1; acetone:MeOH=3:1.
IR [cm⁻¹]: 3338, 3070, 3500-2500, 2968, 2934, 2876, 1724, 1649, 1600, 1391, 1387, 1241, 914.
¹H NMR (CDCl₃): δ (ppm)=12.5 (br.s, 1H, OH), 7.17 (d, J=8.7 Hz, 1H, Ar-H), 6.73 (d, J= 2.7 Hz, 1H, Ar-H), 6.49 (dd, J= 2.7, 8.7 Hz, 1H, Ar-H), 4.46 (dd, J= 4.9 Hz, 8.9 Hz, 1H, CHCH(CH₃)₂), 3.85 (q, J=7.0, 17.0 Hz 1H, CHCH₃), 2.18 (m, 1H, CHCH(CH₃)₂), 1.49 (d, J=7.0 Hz, 3H, CHCH₃), 0.92 (6H, 2×CH₃).
¹³C NMR (CDCl₃): 17.7 (17.3) (CHCH₃), 19.2 (19.4) (2×CH₃), 31.0 (38.6) (CHCH(CH₃)₂), 54.5 (CHCH(CH₃)₂), 56.9 (56.7) (CHCH₃), 113.4 (113.2) (Ar), 114.9 (114.6) (Ar), 120.5 (120.4) (C-Cl), 130.5 (Ar), 132.6 (132.4) (C-Cl), 146.7 (146.5) (CNHCH), 174.0 (173.7) (C=O), 173.5 (173.2) (C=O).

### Example 3c N-[N-(3,4-dichlorophenyl)-D,L-alanyl]-L-leucine

The obtained raw product is oil that gives an amorphous product when treated with diethyl/petroleum ether. Weight 0,95 g m. p. 125-135 °C, yield 98,8 %, chromatographically pure in systems CHCl₃:MeOH=9:1, n-BuOH:AcOH:H₂O= 3:1:1; EtOAc:hexane=1:1; acetone:MeOH=3:1. [α]²²₅₄₆ -58 (c1 MeOH).
IR [cm⁻¹]: 3371, 3341, 3070, 3030-2440, 2960, 2933, 2873, 1719, 1621, 1600, 1389, 1370, 1240,914.
¹H NMR (DMSO): δ (ppm)=12.55 (br.s, 1H, OH), 8.30 (d, J=8.4 Hz, 1H, NH), 7.20 (d, J= 8.8 Hz, 1H, Ar-H), 6.68 (d, J= 2.7 Hz, 1H, Ar-H), 6.56 (dd, J= 2.7, 8.8 Hz, 1H, Ar-H), 4.24 (m, 1H, CHCH₂), 3.90 (q, J=6.8, 1H, CHCH₃), 1.53 (m, 3H, CH₂CH(CH₃)₂, CH₂CH(CH₃)₂), 1.27 (d, J=6.8 Hz, 3H, CHCH₃), 0.80 (6H, 2×CH₃).
¹³C NMR (DMSO): 18.5 (18.7) (CH₃), 20.9 (21.0) (CH₃), 23.0 (22.9) (CH₃), 24.3 (CH₂CH (CH₃)₂), 39.9 (CH₂CH(CH₃)₂), 49.9 (CHCH₂), 51.7 (CHCH₃), 112.0-132.0 (Ar), 148.1 (CNHCH), 174.0 (C=O), 173.3 (C=O).

### Example 3d N-[N-(3,4-dichlorophenyl)-D,L-alanyl]-glycine

The obtained raw product is oil that gives an amorphous product when treated with diethyl ether. Weight 1,17 g m. p. 138-152 °C, yield 69,2 %, chromatographically pure in systems CHCl₃:MeOH=9:1, n-BuOH:AcOH:H₂O= 3:1:1; EtOAc:hexane=1:1; acetone:MeOH=3:1.
[a]²²₅₄₆ +10 (c1 MeOH).
IR [cm⁻¹]: 3393, 3374, 3030-2440, 2984, 2934, 2856, 1740, 1634, 1600, 1214, 935. ¹H NMR (DMSO): δ (ppm)=12.55 (br.s, 1H, OH), 8.28 (t, J=5.8 Hz, 1H, NH), 7.23 (d, J=8.8 Hz, 1H, Ar-H), 6.72 (d, J= 2.6 Hz, 1H, Ar-H), 6.54 (dd, J= 2.6, 8.8 Hz, 1H, Ar-H), 3.85 (q, J=6.9 Hz, 1H, CHCH₃), 3.74 (d, J=5.9, 2H, CH₂).
¹³C NMR (DMSO): 18.8 (CH₃), 40.6 (CH₂), 52.3 (CHCH₃), 113.4 (Ar), 113.1 (Ar), 117.1 (C-Cl), 130.3 (Ar), 131.2 (C-Cl), 148.0 (CNHCH₂), 171.2 (C=O), 173.8 (C=O).

### Example 4. Synthesis of Boc-Gly-OCH₂CN

To a solution of Boc-Gly-OH (1.75g 10.0000 mmol) in DMF were added 2.73-fold excess of chloracetonitrile (2,5 ml 27,3000mmol) and 6.55-fold excess of Et₃N (9,20 ml 65,4545 mmol). The reaction mixture was stirred at room temperature for 24 hours. At the end of the reaction, to the reaction mixture was added 1N HCl and the non-reacted starting products were extracted with EtOAc. The water-acid phase was alkalized using 5 % NaHCO₃ to pH=8, then was extracted again with EtOAc. The organic phase was washed in succession with 5 % NaHCO₃, H₂O, 2N HCl and H₂O till a negative reaction for chlorine ions; dried over Na₂SO₄ and the solvent was distilled under vacuum. A crystalline product is obtained with an yield of 85,0 %, chromatographically pure in a system of CHCl₃:MeOH=9:1 and n-BuOH:AcOH:H₂O= 3:1:1.

### Example 5. Synthesis of 6-O-(Boc-glycyl)-galanthamine

To a solution of galanthamine (0.41 g 1.4273 mmol) in 0.8 ml DMF were added Boc-Gly-OCH₂CN (0.60 g 2.8037 mmol) and DBU (0.11 ml 0.7370 mmol). The reaction mixture was stirred at room temperature while monitoring the depletion of starting components by chromatography in a system of n-BuOH:AcOH:H₂O= 3:1:1. At the end of reaction, to the reaction mixture was added 1N HCl and the non-reacted Boc-Gly-O-CH₂CN was extracted using EtOAc. The water-acid phase was alkalized with 5 % NaHCO₃ to pH=8, then the product obtained was extracted using EtOAc. The organic phase was washed with 5 % NaHCO₃, H₂O, dried over Na₂SO₄ and the solvent was distilled under vacuum.
The obtained product is oil with an yield of 80,1 %, chromatographically pure in a system of CHCl₃:MeOH=9:1 and n-BuOH:AcOH:H₂O= 3:1:1.
IR [cm⁻¹]: 3418 2976, 2933, 2802, 1736, 1705, 1283, 1232, 1205, 1048.

Example 6 Synthesis of 6-O-N-[N-(3,4-dichlorophenyl)-D,L-alanyl]-glycyl-galanthamine To a solution of galanthamine (0.29 g 1.0095 mmol) in DMF was added while stirring N-(3,4-dichlorophenyl)-alanyl-glycin (1.00 g 3.4347 mmol) and upon cooling were added diisopropyl carbodiimide (0.56 ml 3.6165mmol) and DMAP (80 mg). The reaction mixture was stirred at room temperature while monitoring the depletion of starting components by chromatography in a system of n-BuOH:AcOH:H₂O= 3:1:1. At the end of reaction, to the reaction mixture was added 1N HCl and the non-reacted products was extracted using EtOAc. The water-acid phase was alkalized with 5 % NaHCO₃ to pH=8, then extracted again using EtOAc. The organic phase was washed with 5 % NaHCO₃, H₂O, dried over Na₂SO₄ and the solvent was distilled under vacuum.
The obtained raw product is oil that produces amorphous crystals when treated with EtOAc/diethyl ether/petroleum ether. Yield 80,1 %, chromatographically pure in a system of CHCl₃:MeOH=9:1 and n-BuOH:AcOH:H₂O= 3:1:1.
HPLC: Rt= 7.24min.
IR [cm⁻¹] : 3340, 2969, 2933, 2839, 1742, 1664, 1281, 1232, 1196, 1046-1018.
¹H NMR (CDCl₃): δ (ppm)= 7.15(2H, H_{b}, NH), 6.70 (4H, H₁₀'+H₁₁'+Hₐ), 6.45 (2H, H_{c}), 6.35 (dd, 1H), 6.25 (1H, 5'), 5.70-6.0 (1H, 4'), 5.30 (1H, H3'), 4.57 (1H, H1'), 4.30 (1H, H9ₐ'), 4.00 (2H, H1"), 3.87 (3H, OCH₃), 3.80 (3H, H3"+H9_{b}'+H1_{b}"), 3.45 (2H, Hₐ'), 3.20 (1H, H8_{b}'), 2.67 (br.d, H2ₐ'), 2.43 (3H, NCH₃), 2.10 (2H, H2_{b}'+H7ₐ'), 1.65 (1H, H7_{b}'), 1.45 (3H, H3ₐ").

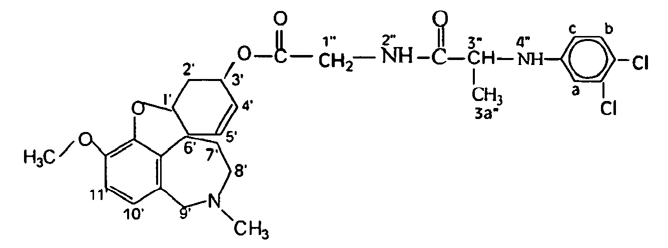

Elemental analysis - determination of nitrogen: C₂₈H₃₂N₃Cl₂O₅, M.M= 560.43 calculated 7.50 % N, found 7.53 % N.
Mass spectrometry: C₂₈H₃₂N₃Cl₂O₅, M.M= 559,2 found[M+H]⁺=560.53.

### Example 7. Synthesis of derivatives of 6-O-(Boc-glycyl)-galanthamine

Solution of 6-O-(Boc-glycyl)-galanthamine (0.25 g 0.5627 mmol) in 1.5 ml TFA was stirred in a round-bottom flask equipped with a chlorine-calcium tube at room temperature. Deblocking Boc-protekting group was monitored by chromatography in systems CHCl₃:MeOH=9:1 and n-BuOH:AcOH:H₂O= 3:1:1. After the end of reaction, TFA was removed under vacuum and the trifluoracetyl salt was treated using diethyl ether. An oil-like raw product was obtained, which was dissolved in DMF, and after cooling Et₃N was added to pH=8. Afterwards, while stirring, dipeptide, derivative of N-(3,4-dichlorophenyl)-D,L-alanine (0,26 g 0.6817 mmol), 0,36 g (0,9491 mmol) of HBTU and 0,14 ml (0.9960 mmol) of Et₃N were added. The reaction mixture was stirred at room temperature while monitoring the depletion of starting 6-O-(H-glycyl)-galanthamine by chromatography in systems of CHCl₃:MeOH=9:1 and n-BuOH:AcOH:H₂O= 3:1:1. At the end of reaction, to the reaction mixture was added 5 % NaHCO₃, which resulted in precipitate formation. It was filtered, and then washed by successive suspending in 5 % NaHCO₃ and H₂O. The obtained raw products were re-crystallized using EtOAc/diethyl ether/petroleum ether or DMF/H₂O.

### Example 7a 6-O-N-[N-(3,4-dichlorophenyl)-D,L-alanyl]-L-phenylalanyl-glycyl-galanthamine

The obtained product is crystalline. M. p. 107-112°C, yield 45,0 %, chromatographically pure in a system of CHCl₃:MeOH=9:1 and n-BuOH:AcOH:H₂O= 3:1:1.
HPLC: Rt= 8.94, 9.35 min. for S and R isomers.
IR [cm⁻¹]: 3335, 3064, 3030, 2932, 2799, 1741, 1661, 1599, 1282, 1232, 1201, 1047, 746, 701.
¹H NMR (CDCl₃): δ (ppm)= 7.50-6.80 (27H), 6.55-6.80 (3H, H₁₀'+H₁₁'), 6.50 (d, 1H, Hₐ), 6.30 (1H, H_{c}), 6.10 (d, 1H, H5'), 5.95 (dd, 1H, H4'), 5.30 (1H, H3'), 4.67 (ddd, 1H, H3), 4.50 (1H, H1'), 4.36 (1H, H9ₐ'), 4.10-3.70 (9H, OCH₃+H9_{b}'+H5+H1ₐ+H1_{b}), 3.50 (1H, H8ₐ'), 3.40-2.80 (5H, H8_{b}'+CH₂-Ph), 2.65 (d, 1H, H2ₐ'), 2.48 (s, 3H, NCH₃), 2.05 (2H, H2_{b}'+H7ₐ'), 1.75 (1H, H7_{b}'), 1.38 (d, 3H, CHCH₃).

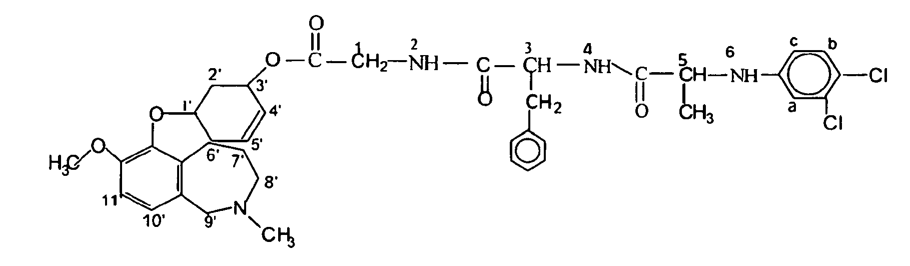

Elemental analysis - nitrogen determination: C₃₇H₄₁N₄Cl₂O₆, M.M= 707.61 calculated 7.92 % N, found 7.94 % N.
Mass spectrometry: C₃₇H₄₁N₄Cl₂O₆, M.M= 706.20, found [M+H]⁺=707.54.

### Example 7b 6-O-N-[N-(3,4-dichlorophenyl)-D,L-alanyl]-L-valyl-glycyl-galanthamine

The obtained product is crystalline. M. p. 143-147°C, yield 51.0 %, chromatographically pure in a system of CHCl₃:MeOH=9:1 and n-BuOH:AcOH:H₂O= 3:1:1.
HPLC:Rt= 7.27 and 7.66 min. for S and R isomers.
IR [cm⁻¹]: 3322, 3069, 3058), 2965, 2933, 1742, 1656, 1599, 1382, 1376, 1283, 1231, 1199, 1047
¹H NMR (CDCl₃): δ (ppm)= 7.20 (2H, H5, NH), 6.68 (4H, H2+H10'+H11'), 6.45 (1H, H6), 6.31 (d, 1H, J=10.3 Hz, H5'), 5.90 (dd, 1H, J=10.3, 4.6 Hz, H4'), 5.35 (1H, H3'), 4.54 (1H, H1'), 4.30 (1H, H3"), 4.14 (d, 1H, J=15.2 Hz, H9ₐ'), 4.10-3.90 (m, 1H, H1ₐ"), 3.85 (4H, OCH₃, H5"), 3.70 (d, 1H, J=15.2 Hz, H9_{b}'), 3.32 (dd, 1H, J=14.0, 12.9 Hz, H8ₐ'), 3.07 (br.d, 1H, J=14.8 Hz, H8_{b}'), 2.66 (br.d, 1H, J=15.8 Hz, H2ₐ'), 2.40 (5H, NCH₃), 2.10 (3H, H2_{b}'+H7ₐ'+H3"-CH), 1.58 (d, 1H, J=13.8 Hz, H7_{b}'), 1.49 (dd, J=6.9, 1.2 Hz, 3H, H5", CHCH₃), 0.8 (m, 6H, 2.CH₃).

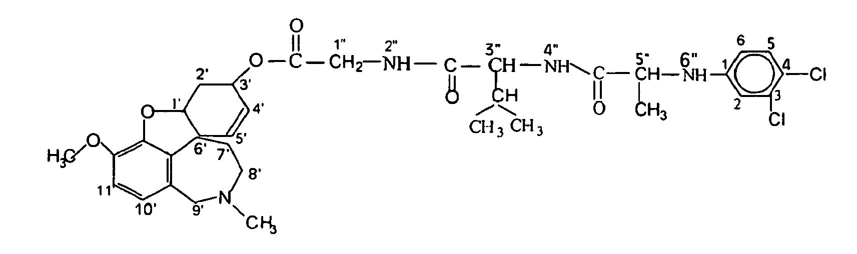

Elemental analysis - nitrogen determination: C₃₃H₄₁N₄Cl₂O₆, M.M= 659.62 calculated 8.50 % N, found 8.59 % N.
Mass spectrometry: C₃₃H₄₁N₄Cl₂O₆, M.M= 658.20 found [M+H]⁺=659.53.

### Example 7c 6-O-N-[N-(3,4-dichlorophenyl)-D,L-alanyl]-L-leucyl-glycyl-galanthamine

The obtained product is crystalline. M. p. 120-125°C, yield 68.0 %, chromatographically pure in a system of CHCl₃:MeOH=9:1 and n-BuOH:AcOH:H₂O= 3:1:1.
HPLC:Rt= 8.66 and 8.97 min for S and R isomers.
IR [cm⁻¹]: 3325, 3068, 3058, 2957, 2934, 2872, 1741, 1657, 1599, 1384, 1378, 1282, 1232, 1199, 1046.
¹H NMR (CDCl₃): δ (ppm)= 7.17 (1H, H5), 7.05 (1H, H2"), 6.66 (5H, H10'+H11'+H2, 2.NH), 6.45 (m, 1H, H6), 6.24 (d, J=10.4 Hz, 1H, H5'), 5.90 (dd, 1H, J= 4.87, 10.0 Hz, H4'), 5.31 (m, 1H, H3'), 4.55 (m, 2H, H1'+H3"), 4.25 (2H, H9a', NH), 3.95 (1H, H1"), 3.87 (s, 3H, OCH₃), 3.85 (2H, H5"+H9_{b}'), 3.45 (m, 1H, H8ₐ'), 3.15 (d, 1H, J=11.9 Hz, H8_{b}'), 2.65 (d, 1H, J= 15.1 Hz, H2ₐ'), 2.46 (s, 3H, NCH₃), 2.10 (m, 2H, H2_{b}'+H7ₐ'), 1.80-1.30 (5H, H7_{b}'+CH₂CH), 1.48 (d, 3H, J=6.9 Hz, H5", CHCH₃),0.8 (6H, 2.CH₃).

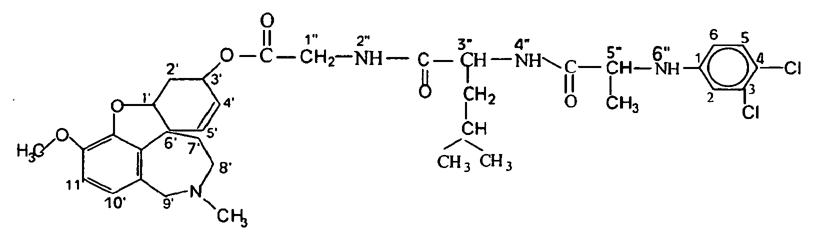

Elemental analysis - nitrogen determination: C₃₄H₄₃N₄ClO₆, M.M= 673.64 calculated 8.32 % N, found 8.40 % N.
Mass spectrometry: C₃₄H₄₃N₄Cl₂O₆, M.M= 672,20 found [M+H]⁺=673.56.

### Example 8. Synthesis of tripeptide-esters, derivatives of N- (3,4-dichlorophenyl)-D,L-alanine

Z-Leu-Gly-OEt, Z-Val-Gly-OEt or Z-Phe-Gly-OEt (10.0000 mmol) was dissolved in a 10-fold excess of HBr/AcOH (100.0000 mmol) and stirred for 30 min. in a round bottom flask equipped with a chlorine-calcium tube. The complete removal of Z-group was monitored by chromatography in a system of n-BuOH:AcOH:H₂O= 3:1:1. After reaction completion and evaporation of HBr/AcOH under vacuum the obtained oil was treated with 3 fold excess of ether. To the residue was added DMF and under cooling was added Et₃N to pH=8. Then N-(3,4-dichlorophenyl)-D,L-alanine (13.0000 mmol), HBTU (12.0000 mmol) and Et₃N (12.0000 mmol) were added. The reaction mixture was stirred at room temperature for 24 hours, then was added to it H₂O. The product was extracted using EtOAc and the organic phase was washed in succession with 5 % NaHCO₃, H₂O, 2N HCl and H₂O till a negative reaction for chlorine ions; dried over Na₂SO₄ and the solvent was distilled under vacuum. The obtained products are amorphous. Yield (70-80%). Chromatographically pure in system of CHCl₃:MeOH=9:1 and n-BuOH:AcOH:H₂O= 3:1:1.

### Example 9 Hydrolysis of tripeptide esters to tripeptide-free acids derivatives of N- (3,4-dichlorophenyl)-D,L-alanine

To a solution of the tripeptide esters obtained under Example 8, derivative of N- (3,4-dichlorophenyl)-D,L-alanine in MeOH 4-fold excess of 2N NaOH was added. The reaction mixture was stirred at room temperature and the depletion of starting components was monitored by means of TLC in a system of EtOAc:hexane=1:1. The reaction was terminated upon ester depletion and after acidifying to pH=7, MeOH was removed under vacuum. The water-alkaline phase was acidified using 2N HCl to pH=2-3, then extracted with EtOAc. The product was extracted from the organic phase using 5 % NaHCO₃, then the water-alkaline phase was acidified again with 2N HCl to pH=2-3 and extracted again using EtOAc, washed with H₂O to a negative reaction for chlorine ions; dried over Na₂SO₄ and the solvent was distilled under vacuum. The obtained raw products are oils that produce amorphous crystals when treated with diethyl ether/petroleum ether with almost quantitative yields. Chromatographically pure in systems of CHCl₃:MeOH=9:1, n-BuOH:AcOH:H₂O= 3:1:1 and EtOAc:hexane=1:1.

### Example 10. General method for synthesis of galanthamine derivatives.

To a solution of galanthamine (0.30 g 1.0094 mmol) in DMF under cooling were added tripeptide-free acids, derivative of N- (3,4-dichlorophenyl)-D,L-alanine (3.4347 mmol), diisopropyl carbodiimide (3.6165 mmol) and DMAP (0.1 mmol). The reaction mixture was stirred for 24 hours at room temperature and the end of the reaction was monitored in a system of n-BuOH:AcOH:H₂O= 3:1:1 till galanthamine depletion. To the reaction mixture was added 5 % NaHCO₃ resulting in precipitate formation. The precipitate was washed by suspending in 5 % NaHCO₃ and H₂O. After drying the residue was re-crystallized from EtOAc/diethyl ether/petroleum ether or DMF/H₂O. Then multiple treatment with diethyl ether to almost complete depletion of diisopropyl carbodiimide was performed, which is monitored by chromatography in a system of n-BuOH:AcOH:H₂O= 3:1:1. Yields 45.0-55.0 %.

### Example 11. Preparation of norgalanthamine

To a solution of galanthamine (2.88 g 10.0200 mmol) in CHCl3 (17 ml) were added in portions within 30 to 50 min m-chloroperbenzoic acid (2.02 g 11.7300 mmol), so that the solution temperature to be between 15° and 50 °C. After stirring for 1-2 hours, the reaction mixture was cooled to 0-10 °C. A solution of FeSO₄.7H₂O (0.28 g 1.0000 mmol) in H₂O (10 ml) was added within 40-50 min so as to maintain the temperature between 0-10 °C. After addition of FeSO₄.7H₂O the reaction mixture was stirred for another 10 min at 0-10 °C and HCl (1.44 ml) was added. 2/3 of the solvent was distilled under vacuum. The reaction mixture was washed with diethyl ether at 30-40 °C to separate the m-chlorobenzoic acid produced as a result of the reaction. The water phase was stirred for 5 to 24 hours at 0-10 °C under chromatographic control. The solvent was distilled under vacuum and the obtained oil was dried under vacuum at 25 °C. An amorphous substance is obtained with purity monitored by HPLC, yield 97,0 %.
HPLC: Rt= 3.7 min.
IR [cm⁻¹]: 3545, 3414, 3239, 2940, 2916, 2839, 2718, 2647, 2596, 2549, 1656, 1628, 1609, 1592, 1280, 1209, 1085, 1045.
¹H NMR (DMSO): 8.20 (br.s., 4H, OH, NH), 6.06 (d, 1H, J=10.3 Hz, H5), 5.87 (dd, J=10.2, 4.5 Hz, H4), 4.53 (br.s, 1H, H1), 4,44 (d,1H, J=5.1 Hz, H9a), 4.22 (d, 1H, J=5.1 Hz, H9b), 3,75 (s, 3H, OCH₃), 3,65 (s, 0.5H), 3,50 (m, 2H, H8a,b), 2,30 (br.d, 1H, J= 15.66 Hz, H2a), 2,05 (m, 2H, H2b+H7a), 1,86 (br.d, 1H, J=15.6 Hz, H7b)
¹³C NMR (dept 135): 129.8 (C5), 125.8 (C4), 122.2 (C11), 112.0 (C12), 87.0 (C1), 60.1 (C3), 56.0 (CH₃O), 50.0 (C9), 44.8 (C8), 34.8 (C7), 31.0 (C2).

### Example 12. Synthesis of 11-N-demethyl-11-N-N-[N-(3,4-dichlorophenyl)-D,L-alanyl]-L-phenylalanyl- galanthamine

To a solution of norgalanthamine hydrochloride (0.37 g 1.1991 mmol) in DMF under cooling Et₃N (0.17 ml 1.2094 mmol) and N-(3,4-dichlorophenyl)-alanyl-phenylalanine (0.50 g 1.3117 mmol) and N-cyclohexyl-N-[β-(N-methylformolino)-ethyl-] carbodiimide (0.56 g 1.3221 mmol) were added. The reaction mixture was stirred for 24 hours at room temperature and the end of reaction was monitored in a system of n-BuOH:AcOH:H₂O= 3:1:1 till norgalanthamine depletion. To the reaction mixture 5 % NaHCO₃ was add, followed by precipitate formation. The precipitate was washed by suspending in 5 % NaHCO₃ and H₂O. After drying, recrystallization with EtOAc/diethyl ether/petroleum ether or DMF/H₂O was performed.

### Example 13. Synthesis of 11-N-demethyl-11-N-N-[N- (3,4-dichlorophenyl)-D,L-alanyl]-L-valyl- galanthamine

To a solution of norgalanthamine hydrochloride (0.42 g 1.3560 mmol) in DMF under cooling Et₃N (0.19 ml 1.3520 mmol) and N-(3,4-dichlorophenyl)-alanyl-valine (0.50 g 1.5009 mmol) and N-cyclohexyl-N-[β-(N-methylformolino)-ethyl-] carbodiimide (0.64 g 1.5109 mmol) was added. The reaction mixture was stirred for 24 hours at room temperature and the end of reaction was monitored in a system ofn-BuOH:AcOH:H₂O= 3:1:1 To the reaction mixture 5 % NaHCO₃ was add, followed by precipitate formation. The precipitate was washed by suspending in 5 % NaHCO₃ and H₂O. The product was dried and recrystallized with EtOAc/diethyl ether/petroleum ether or DMF/H₂O.

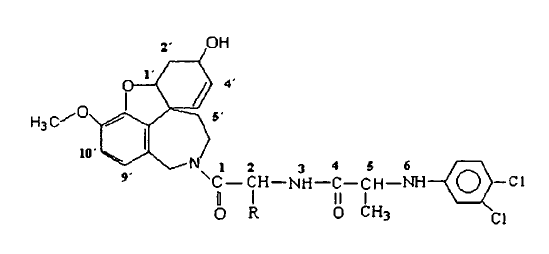

11-N-demethyl-11-N-N-[N-(3,4-dichlorophenyl)-D,L-alanyl]-L-phenylalanyl- galanthamine M. p. 137-141 °C, yield 37 %, chromatographically pure in a system of CHCl₃:MeOH=9:1 and n-BuOH:AcOH:H₂O= 3:1:1.
IR [cm-¹]: 3384, 3062, 3028, 2930, 1640, 1599, 1280, 1240, 1059, 753, 701.
HPLC: Rt= 2.58 and 2.76 min for S and R isomers.
1H NMR (CDCl₃, ppm) δ: 7.10-7.30 (2H, NH), 7.0-6.20 (10H, 5H Ph + H9', H10'+ 3H PhCl), 6.0 (m, 2H, H4', H5'), 4.7-4.4 (m, 4H, CH₂NCH₂), 4.5 (m, 1H, H1'), 4.14 (m, 1H, H3'), 3.85 (s, 3H, O-CH₃), 3.90 (m, 1H, CHCH₂Ph), 3.75 (q, 1H, CHCH₃), 3.20 (m, 1H, CHCH₂Ph), 2.70 (d, J=15.9 Hz, 1H, H2a'), 2.02 (d, J=15.9 Hz, 1H, H2b'), 1.90 (m, CH2), 1.30 (d, , 3H, CHCH₃).
Elemental analysis - nitrogen determination: C₃₄H₃₆N₃Cl₂O₅, M.M= 636.54 theoretical 6.60 % N, calculated 6.62 % N.
Mass spectrometry: C₃₄H₃₆N₃Cl₂O₅ M.M= 636.20 found [M+H]⁺=636.55.
11-N-demethyl-11-N-N-[N- (3,4-dichlorophenyl)-D,L-alanyl]-L-valyl- galanthamine M.p. 133-138 °C, yield 35 %, chromatographically pure in a system of CHCl₃:MeOH=9:1 and n-BuOH:AcOH:H₂O= 3:1:1.
IR [cm⁻¹]:3338, 2963, 2931, 2874, 1656, 1630, 1599, 1382, 1373, 1279, 1214, 1059.
HPLC: Rt= 2.35 and 2.53 min for S and R isomers.
1H NMR (CDCl₃, ppm) δ: 7.10-7.30 (2H, NH), 7.0-6.20 (5H, H9', H10', 3H PhCl), 5.90 (m, 2H, H4', H5'), 4.7-4.4 (m, 4H, CH₂NCH₂), 4.5 (m, 1H, H1'), 4.15 (m, 1H, H3'), 3.86 (s, 3H, O-CH₃), 3.85 (m, 1H, H2), 3.75 (q, 1H, CHCH₃), 3.20 (m, 1H), 2.70 (d, J=15.9 Hz, 1H, H2a'), 2.40 (m, 1H, CH(CH₃)₂), 2.02 (d, J=15.9 Hz, 1H, H2b'), 1.90 (m, CH₂), 1.30 (d, 3H, CHCH₃), 0.80 (d, 6H, 2 x CH₃).
Elemental analysis - nitrogen determination: C₂₉H₃₃N₃Cl₃O₅, M.M= 588.41 calculated 7.14 % N, found 6.98 % N.
Mass spectrometry: C₂₉H₃₃N₃Cl₂O₅, M.M= 587.20 found [M+H]⁺=588.45.

Example 14 The study of inhibitory effect on γ-secretase of the compounds under the inventions took place on expressing APP751 HEK293 cells using the following methods: (1) cell cultivation of expressing APP751 HEK293 cells, and (2) ELISA (solid-phase sandwich enzyme-linked immunosorbent assay) of human β-amyloid protein.
HEK293 cells were cultivated in Dulbecco's modified Eagle's medium (Applichem, Germany) with added 10% fetal calf serum (Biowhittaker, USA) and 200 U/ml Penicillin and 200 mg/ml Streptomycin (Applichem, Germany) at 37°C and 5% CO₂.
ELISA kits of human β-amyloid are intended for in vitro quantitative determination of a native or recombinant β-amyloid in serum, supernatant of cell cultures or other biological samples. In short, a monoclonal antibody specific for the amino end of β-amyloid was immobilized for a 96-pit plate. β-amyloid antigen from the standards or from the samples was connected with this first antibody. Then a second antibody was placed for detection of the specific β-amyloid 1 - 42. An anti-rabbit antibody IgG-HRP completed the sandwich. The reaction was visualized by means of 3,3",5,5"-tetramethylbenzidine (TMB) substrate, following by a stop solution. Yellow colour intensity was in direct proportion to the concentration of β-amyloid in the original sample. The sensitivity of these ELISA was < 10 pg/ml β-amyloid.

ELISA method was used to study the inhibitory effect on γ-secretase of the compounds obtained according to the invention on expressing APP751 HEK293 cells. The following abbreviations were used:
1. 6-O-N-[N- (3,4-dichlorophenyl)-D,L-alanyl]-glycyl galanthamine - AG-GAL
2. 6-O-N-[N-(3,4-dichlorophenyl)-D,L-alanyl]-L-phenylalanyl-glycyl galanthamine - AFG-GAL
3. 6-O-N-[N- (3,4-dichlorophenyl)-D,L-alanyl]-L-leucyl-glycyl-galanthamine - ALG-GAL
4. 6-O-N-[N- (3,4-dichlorophenyl)-D,L-alanyl]-L-valyl-glycyl-galanthamine - AVG-GAL
5. N-(3,4-dichlorophenyl)-D,L-alanyl-L-phenylalanine - AF
6. N-(3,4-dichlorophenyl)-D,L-alanyl-L-leucine - AL.

ELISA specific for human amyloid β was used to determine the possible inhibitory effect of these substances on γ-secretase. The ELISA kits used determined such β-amyloid (1-x) variants that were split off the C-end for any reason. With these ELISA kits, variants of β-amyloid shorter than Aβ (1-16) could not be determined. The first immobilized antibody was specific for the N-end. Thus, using this ELISA kit, both Aβ (1-40) and Aβ (1-42) could be determined. DAPT was used as a control inhibitor. Its effect of γ-secretase inhibitor was judged by the decrease β amyloid quantity in the cell medium. In all trials described below, the stable expressing APP751 HEK293 cells were incubated for 6 hours in a cell medium not containing calf fetal serum. As visible from figure 1, DAPT decreases the quantity of β amyloid by inhibiting γ-secretase activity in concentrations higher than 160 nM. In this trial formulation, 1 x 10⁶ cells were used for 1 pit of the 24-pit plate. To determine the possible inhibition of γ-secretase, the substances listed above were used in concentrations from 0,02, 0,2 and 2 µM.

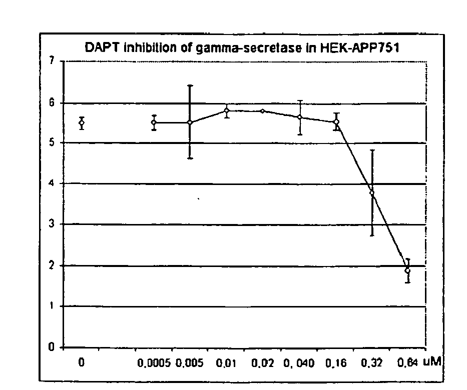

Figure 1: Effect of γ-secretase inhibitor DAPT on the quantity of amyloid β. DAPT was used in concentration of 0,0005, 0,005, 0,010, 0,020, 0,040, 0,160, 0,320, 0,64 µM. The cells were incubated in pit of 24-pit plates for 6 hours. ELISA of amyloid β (1-x).

During the trial, two of the substances (Figure 2), AVG-GAL and AL showed a significant decrease of β-amyloid quantity in the cell medium. These substances have γ-secretase activity in concentrations higher than 0,02 µM.

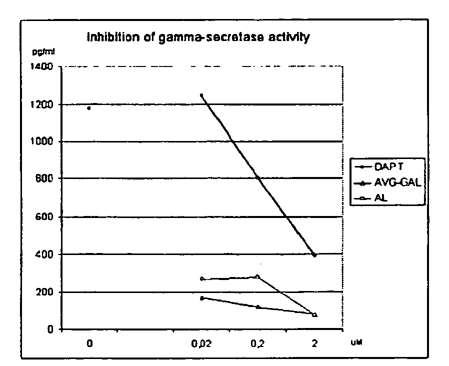

Figure 2: Effect of AVG-GAL and AL on the quantity of amyloid β. The substances were used in concentrations of 0,020, 0,2 and 2 µM. The cells were incubated in I pit of 24-pit plates for 6 hours. ELISA of amyloid β (1-x).

As shown in figure 3, in a concentration of 0,02 µM AG-GAL and ALG-GAL decreased significantly the quantity of amyloid β. γ-Secretase effect of AF and ALG-GAL was comparable with the effect of DAPT in concentrations of 2 µM. In this concentration AG-GAL had no inhibitory effect.

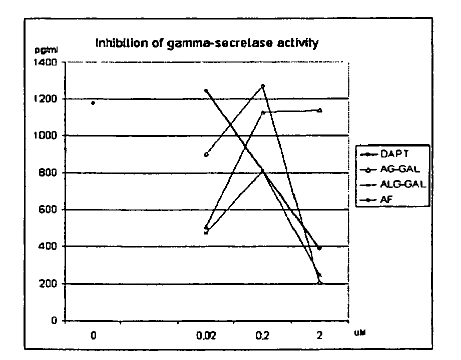

Figure 3: Effect of AG-GAL, ALG-GAL and AF on the quantity of amyloid β. The substances were used in concentrations of 0,020, 0,2 and 2 µM. The cells were incubated in 1 pit of 24-pit plates for 6 hours. ELISA of amyloid β (1-x).

### Example 15 Inhibitory effect of galanthamine derivatives with respect to acetylcholinesterase

In studying the inhibitory effect of the compounds according to the invention, a modified Elman's method was used. Acetylcholine iodide was used as substrate. Acetylcholinesterase is a commercial product (Sigma). Enzyme activity determination was performed in 0,1 M sodium phosphate buffer pH 8,0 at 25 °C. To 1 ml of the buffer, 90 mcl (7,5 MM) acetylcholine iodide and 45 mcl (10 MM) 5,5'-dithiobis-2-nitrobenzoic acid (DTNB) were added. After stirring, 1 EU acetylcholinesterase was added. The enzyme reaction was monitored according to the change of absorption at 412 nm in a period of 2 to 10 min. The speed of the enzyme reaction was calculated in M/l.sec, using an absorption coefficient of 14,15.10³ 1/M.cm ( Eyer ). Each test was repeated 3 times. The test substances was dissolved in 50% ethyl alcohol.

To trace the inhibiting effect, the test compounds were added in different concentrations to 1,5 ml of phosphate buffer and 1 EU enzyme. They were incubated for 30 min at 25 °C, then acetylcholine iodide and DTNB were added and the course of the enzyme reaction was monitored according to the description above. The percentage of enzyme activity inhibition was determined against a control that does not contain the test substances. After a preliminary screening, such concentrations of the test substances was selected, that have an inhibiting effect between 20 and 80%.

To determine the inhibition constant (Ki), minimum 3 different concentrations of the compounds under study were used. The inhibition curves were made for each selected concentration of the inhibitor using the method of double reciprocal values (Lineweaver, Burk), the substrate concentration being within 90 - 3300 .10⁻⁶ M/l.
IC₅₀ (inhibitor concentration required for 50% inhibition of enzyme activity) were determined using the log-probit method. The calculations were made using the matlab6 program.
IC₅₀ values were determined on the basis of experimental data obtained for different concentrations of the compounds manifesting inhibitory activity.

| Name of compound | Molecular mass | IC₅₀ (µM) |
|---|---|---|
| 1. 6 -O-N-[N- (3,4-dichlorophenyl)-D,L-alanyl]-glycyl galanthamine | 560.43 | 3,981 |
| 2.6-O-N-[N-(3,4-dichlorophenyl)-D,L-alanyl]-L-phenylalanyl-glycyl galanthamine | 707.70 | 3,758 |
| 3.6-O-N-[N-(3,4-dichlorophenyl)-D,L-alanyl]-L-leucyl-glycyl galanthamine | 673.40 | 3,35 |
| 4. 6-O-N-[N-(3,4-dichlorophenyl)-D,L-alanyl]-L-valyl-glycyl galanthamine | 659.62 | 8,33 |
| 5. 11-N-demethyl-11-N-N-[N-(3,4-dichlorophenyl)-D,L-alanyl]-L-phenylalanyl- galanthamine | 636.40 | 12,5 |
| 6. 11-N-demethyl-11-N-N-[N-(3,4-dichlorophenyl)-D,L-alanyl]-L-valyl- galanthamine | 588.40 | 31,25 |
| Galanthamine | 287.35 | 5,0 |

### Example 16 Inhibitory effect of galanthamine derivatives with respect to butyrylcholinesterase.

In studying the inhibitory effect of the compounds according to the invention, a modified Elman's method was used. Butyrylcholine iodide was used as substrate. Butyrylcholinesterase is a commercial product (Sigma). Enzyme activity determination was performed in 0,1 M sodium phosphate buffer pH 8,0 at 25°C. To 1 ml of the buffer, 90 mcl (7,5 MM) butyrylcholine iodide and 45 mcl (10 MM) 5,5'-dithiobis-2-nitrobenzoic acid (DTNB) were added. After stirring, 1 EU butyrylcholinesterase was added. The enzyme reaction was monitored according to the change of absorption at 412 nm in a period of 2 to 10 min. The speed of the enzyme reaction was calculated in M/l.sec, using an absorption coefficient of 14,15 .10³ 1/M.cm ( Eyer ). Each test was repeated 3 times. The substances under study were dissolved in 50% ethyl alcohol.

To trace the inhibiting effect, the compounds under study were added in different concentrations to 1,5 ml of phosphate buffer and 1 EU enzyme. They were incubated for 30 min at 25 °C, then butyrylcholine iodide and DTNB were added and the course of the enzyme reaction was monitored according to the description above. The percentage of enzyme activity inhibition was determined against a control that does not contain the test substances. After a preliminary screening, such concentrations of the test substances were selected that have an inhibiting effect between 20 and 80%.

To determine the inhibition constant (Ki), minimum 3 different concentrations of the test compounds under study were used. The inhibition curves for each selected concentration of the inhibitor were made using the method of double reciprocal values (Lineweaver, Burk), substrate concentration being within 90 - 3300 .10⁻⁶ M/l.
IC₅₀ values were determined using the log-probit method. The calculations were made using the matlab6 program.
IC₅₀ values were made on the basis of experimental data obtained for different concentrations of the compounds manifesting inhibitory activity.

| Name of compound | Molecular mass | IC₅₀ (µM) |
|---|---|---|
| 1. 6-O-N-[N- (3,4-dichlorophenyl)-D,L-alanyl]-glycyl galanthamine | 560.43 | 23,71 1 |
| 2. 6-O-N-[N-(3,4-dichlorophenyl)-D,L-alanyl]-L-phenylalanyl-glycyl galanthamine | 707.70 | 10,0 |
| 3. 6-O-N-[N- (3,4-dichlorophenyl)-D,L-alanyl]-L-leucyl-glycyl galanthamine | 673.40 | 0,612 |
| 4. 6-O-N-[N-(3,4-dichlorophenyl)-D,L-alanyl]-L-valyl-glycyl galanthamine | 659.62 | 0,335 |
| 5.11-N-demethyl-11-N-N-[N-(3,4-dichlorophenyl)-D,L-alanyl]-L-phenylalanyl- galanthamine | 636.40 | 0,447 |
| 6.11-N-demethyl-11-N-N-[N-(3,4-dichlorophenyl)-D,L-alanyl]-L-valyl- galanthamine | 588.40 | 0,603 |
| Galanthamine | 287.35 | 59,2 |

## Claims

1. Galanthamine derivatives containing a peptide fragment of general formula I 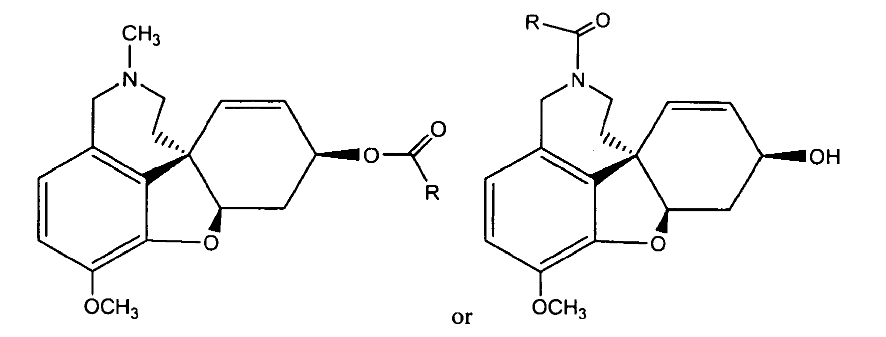 wherein:
R is a dipeptide or a tripeptide that contains in the side chain of aminoacids and/or at its N-end one or more alkyl, aryl, alkylaryl, phenyl, alkylphenyl, alkoxyphenyl residues, and particularly for side chains of aminoacids -CH(CH₃)₂, -CH₂C₆H₅, -CH₂CH(CH₃)₂; and for the N-end phenyl, alkylphenyl, alkoxyphenyl, halogenphenyl, wherein the halogen atoms being one or more Cl, F, Br;
as well as their **diastereoisomers** or their acid addition salts.

2. Method for obtaining galanthamine derivatives **characterized by** the fact that galanthamine or norgalanthamine is condensed with dipeptides or tripeptide free acids, substituted in certain cases, under the effect of a condensing agent and in the presence of a base in a medium of organic solvent.

3. Method for obtaining galanthamine derivatives according to claim 2 **characterized by** the fact that galanthamine or norgalanthamine is condensed with dipeptides or tripeptide free acids, substituted in certain cases, under the effect of a condensing agent selected from the group of diisopropylcarbodiimide, dicyclohexyl carbodiimides and N-cyclohexyl-N-[β-(N-methylmorpholino)-ethyl-] carbodiimide and a base selected from the group of DMAP, Et₃N, Me₃N, pyridine, tributylamine, DIPEA in an organic solvent medium selected from the group of CH₂Cl₂, DMF, CHCl₃, EtOAc, dimethylsulfoxide at room temperature.

4. Method for obtaining galanthamine derivatives **characterized by** the interaction of Boc-Gly-OCH₂CN with galanthamine in the presence of a condensing agent in a medium of minimum quantity of organic solvent; the obtained ester 6-O-(Boc-glycyl)-galanthamine is isolated by acidifying and subsequent removal of non-reacted products by extraction with organic solvent, alkalization of the water-acid phase and repeated extraction of the target product using an organic solvent, followed by deblocking of Boc-protective group with an acid; the obtained ester is condensed using different dipeptides or tripeptide free acids, substituted in certain cases, under the effect of a condensing agent in a medium of organic solvent and in the presence of a base at room temperature; the obtained raw galanthamine derivatives are subjected to recrystallization.

5. Method for obtaining galanthamine derivatives according to Claim 4, **characterized by** the fact that the interaction between galanthamine and Boc-Gly-OCH₂CN takes place in the presence of DBU in a medium of organic solvent selected from the group of CH₂Cl₂, DMF, CHCl₃, EtOAc, dimethylsulphoxide.

6. Method for obtaining galanthamine derivatives according to Claim 4, **characterized by** the fact that the ester 6-O-(Boc-glycyl)-galanthamine is isolated as a chromatographically pure product, by acidifying with dilute HCl and extraction of non-reacted products with an organic solvent selected from the group of CH₂Cl₂, CHCl₃, EtOAc, alkalization of the water-acid phase to pH=8 and subsequent extraction of the target product with an organic solvent selected from the group CH₂Cl₂, CHCl₃, EtOAc.

7. Method for obtaining galanthamine derivatives according to Claim 4, **characterized by** the fact that deblocking of Boc-protective group takes place with an acid selected from the group of TFA, hydrochloric acid/organic solvent, HBr/AcOH and subsequent processing with diethyl ether.

8. Method for obtaining galanthamine derivatives according to Claim 4, **characterized by** the fact that condensing of the ester 6-O-(Boc-glycyl)-gatanthamine with different dipeptide or tripeptide free acids, substituted in certain cases, takes place under the effect of a condensing agent selected from the group of HBTU or TBTU or TCTU in a medium of an organic solvent selected from the group of CH₂Cl₂, DMF, CHCl₃, EtOAc, dimethylsulphoxide, and in the presence of a base selected from the group of Et₃N, Me₃N, pyridine, tributylamine, DIPEA at room temperature.

9. Use of galanthamine derivatives according to Claim 1 for the manufacturing of a product for inhibition of acetylcholinesterase, butyrylcholinesterase and gamma-secretase.

10. Use of a compound of formula I according to Claim 1, for the manufacturing on a product for preventing and/or treating neurodegenerative diseases.

11. Use of a compound of formula I according to Claim 10, for the manufacturing of a product for preventing and/or treating senile dementia and Alzheimer's disease.

12. Pharmaceutical formulation containing a therapeutically active quantity of at least one of the compounds according to Claim 1 and a pharmaceutically acceptable carrier and/or excipients.

## Patentansprüche

1. Galanthamin-Derivate mit Peptidfragment der allgemeinen Formel I 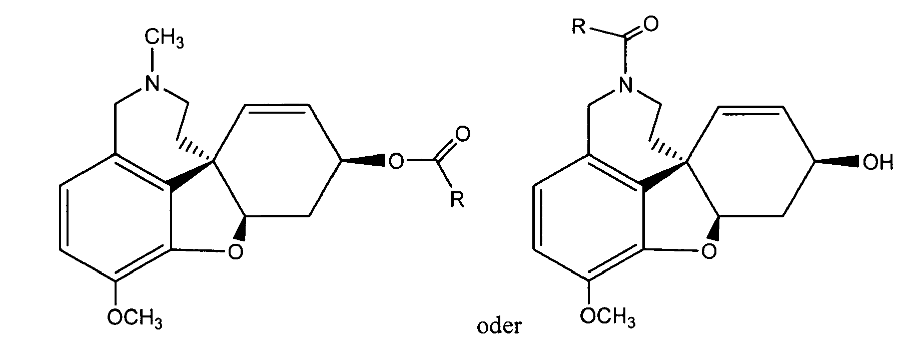 in der die Substituente die unten angegebene Bedeutungen haben:
R ist Dipeptid oder Tripeptid, beinhaltend in der Seitenkette der Aminosäuren und/oder an seinem N-Ende einen oder mehrere Alkyl-, Aryl-, Alkylaryl-, Phenyl-, Alkylphenyl-, Alkoxyphenyl-Reste und insbesondere für die Seitenketten der Aminosäuren -CH(CH₃)₂, - CH₂C₆H₅, -CH₂CH(CH₃)₂; und für das N-Ende Phenyl, Alkylphenyl, Alkoxyphenyl, Halogenphenyl, wobei die Halogenatome eins oder mehrere Cl, F, Br sein können; als auch deren Diastereoisomeren oder deren Säure-Additionssalzen.

2. Verfahren zur Gewinnung von Galanthamin-Derivaten, **dadurch gekennzeichnet, dass** Galanthamin oder Norgalanthamin mit Dipeptiden oder Tripeptiden freie Säuren kondensiert wird, gegebenenfalls substituiert, unter der Wirkung von kondensierender Agenz und in der Gegenwart einer Base im Medium eines organischen Lösungsmittels.

3. Verfahren zur Gewinnung von Galanthamin-Derivaten, nach Anspruch 2, **dadurch gekennzeichnet, dass** Galanthamin oder Norgalanthamin mit Dipeptiden oder Tripeptiden freie Säuren kondensiert wird, gegebenenfalls substituiert, unter der Wirkung von kondensierender Agenz, ausgewählt aus der Gruppe Diisopropylcarbodiimid, Dicyclohexylcarbodiimid und N-Cyclohexyl-N-[β-(N-methylmorpholino)-ethyl-]carbodiimid und einer Base, ausgewählt aus der Gruppe von DMAP, Et₃N, Me₃N, Pyridin, Tributylamin, DIPEA im Medium eines organischen Lösungsmittels, ausgewählt aus der Gruppe von CH₂Cl₂, DMF, CHCl₃, EtOAc, Dimethylsulfoxid bei Raumtemperatur.

4. Verfahren zur Gewinnung von Galanthamin-Derivaten, **dadurch gekennzeichnet, dass** Boc-Gly-OCH₂CN mit Galanthamin zusammenwirkt in Anwesenheit von kondensierender Agenz im Medium von minimaler Menge eines organischen Lösungsmittels; der gewonnene Ester 6-O-(Boc-glycil)-galanthamin wird isoliert durch Versäuerung und anschliessende Entfernung der nicht reagierten Produkte durch Extrahierung mit organischem Lösungsmittel, Alkalisieren der Wasser-Säure-Phase und wiederholte Extrahierung des Zielproduktes mit organischem Lösungsmittel; es folgt eine Deblockierung der Boc-Schutzgruppe mit Säure; der gewonnene Ester wird mit verschiedenen Dipeptiden oder Tripeptiden freie Säuren kondensiert, gegebenenfalls substituiert, unter der Wirkung von kondensierender Agentz im Medium von organischem Lösungsmittel und in Gegenwart einer Base bei Raumtemperatur; und die gewonnenen rohen Galanthamin-Derivate werden einer Rekristallisation unterworfen.

5. Verfahren zur Gewinnung von Galanthamin-Derivaten nach Anspruch 4, **dadurch gekennzeichnet, dass** die Zusammenwirkung von Galanthamin und Boc-Gly-OCH₂CN in Gegenwart von DBU erfolgt im Medium von organischem Lösungsmittel, ausgewählt aus der Gruppe CH₂Cl₂, DMF, CHCl₃, EtOAc, Dimethylsulphoxid.

6. Verfahren zur Gewinnung von Galanthamin-Derivaten nach Anspruch 4, **dadurch gekennzeichnet, dass** der Ester 6-O-(Boc-glycyl)-galanthamin als reines chromatographisches Produkt isoliert wird, durch Versäuerung mit verdünnter HCl und Extraktion der nicht reagierten Produkte mit organischem Lösungsmittel, ausgewählt aus der Gruppe CH₂Cl₂, CHCl₃, EtOAc, Alkalisierung der Wasser-Säure-Phase bis pH=8 und nachfolgende Extrahierung des Zielproduktes mit organischem Lösungsmittel, ausgewählt aus der Gruppe CH₂Cl₂, CHCl₃, EtOAc.

7. Verfahren zur Gewinnung von Galanthamin-Derivaten nach Anspruch 4, **dadurch gekennzeichnet, dass** die Deblockierung der Boc-Schutzgruppe mit Säure durchgeführt wird, ausgewählt aus der Gruppe TFA, Salzäure/organisches Lösungsmittel, HBr/AcOH, und nachfolgender Behandlung mit Diethylester.

8. Verfahren zur Gewinnung von Galanthamin-Derivaten nach Anspruch 4, **dadurch gekennzeichnet, dass** die Kondensation des Esters 6-O-(Boc-glycyl)-galanthamin mit verschiedenen Dipeptiden freie Säuren, gegebenenfalls substituiert, unter der Wirkung von kondensierender Agenz ausgeführt wird, ausgewählt aus der Gruppe HBTU oder TBTU oder TCTU im Medium von organischem Lösungsmittel, ausgewählt aus der Gruppe CH₂Cl₂, DMF, CHCl₃, EtOAc, Dimethylsuphoxid, und in Gegenwart einer Base, ausgewählt aus der Gruppe Et₃N, Me₃N, Pyridin, Tributylamin, DIPEA bei Raumtemperatur.

9. Verwendung von Galanthamin-Derivaten nach Anspuch 1, für die Gewinnung eines Produkts zur Inhibierung der Acetylcholinesterase, der Butylcholinesterase und der Gamma-Sekretase.

10. Verwendung einer Verbindung der Formel I nach Anspruch 1, zur Gewinnung eines Produkts für die Prophylaxe bzw. Behandlung von neurodegenerativen Erkrankungen.

11. Verwendung einer Verbindung der Formel I nach Anspruch 10 zur Gewinnung eines Produkts für die Prophylaxe bzw. die Behandlung der senilen Demenz und der Alzheimerischen Krankheit.

12. Pharmazeutische Zusammensetzung, beinhaltend therapeutisch aktive Menge von wenigstens einer der Verbindungen nach Anspruch 1 und pharmazeutischverträgliche Träger bzw. Hilfsstoffen.

## Revendications

1. Des dérivés de la galanthamine avec fragment de peptide avec une formule générale I 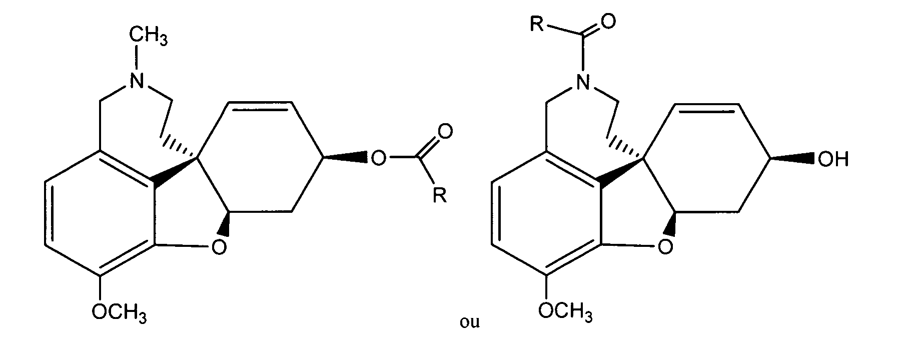 où les éléments remplacés ont les significations données plus bas:
R est dipeptide ou tripeptide, contenant dans le réseau latéral des aminoacides et/ou dans son extrémité N un ou plusieurs résidus d'alkyle, aryle, alkylearyle, phényle, alkylephényle, alcoxyphényle et plus spécialement pour les réseaux latéraux des aminoacides -CH(CH₃)₂, -CH₂C₆H₅, -CH₂CH(CH₃)₂; mais pour l'extrémité N phényle, alkylephényle, alcoxyphényle, halogènephényle, et les atomes halogènes peuvent être un ou plusieurs Cl, F, Br; aussi bien que leurs diastereoisomères et leurs sels acides à annexion.

2. Méthode d'obtention des dérivés de la galanthamine, se caractérisant avec le fait que, la galanthamine ou la norgalanthamine se condense avec des acides libres dipeptides ou tripeptides, dans le cas donné remplacés, sous l'action de l'agent condensant et dans la présence de base dans un milieu de dissolvant organique.

3. Méthode d'obtention des dérivés de la galanthamine, suivant la revendication 2, se caractérisant avec le fait que, la galanthamine ou la norgalanthamine se condense avec des acides libres dipeptides ou tripeptides, dans le cas donné remplacés, sous l'action de l'agent condensant sélectionné du groupe des diisopropylcarbodiimide, dicyclohexylecarbodiimide et N-cyclohexyle-N-[β-(N-méthylemorpholine)-éthyle] carbodiimide et base sélectionnée du groupe des DMAP, Et₃N, Me₃N, pyridine, tributalamine, DIPEA dans un milieu de dissolvant organique sélectionné du groupe CH₂Cl₂, DMF, CHCl₃, EtOAc, dimethylesulfoxide à température de chambre.

4. Méthode d'obtention des dérivés de la galanthamine, se caractérisant avec le fait que, Boc-Gly-OCH₂CN interagit avec la galanthamine dans la présence d'un agent condensant dans un milieu de dissolvant organique en quantité minimale; l'ester obtenu 6-O-(Boc-glicyle)- galanthamine est isolé en commençant par aciduler et ensuite par enlèvement des produits qui n'ont pas réagi par extraction avec un dissolvant organique, alcalisation de la phase hydro-acide et une seconde extraction du produit en objective avec un dissolvent organique; c'est suivi du déblocage du Boc-groupe de protection avec acide; l'ester obtenu se condense avec différents acides libres dipeptides ou tripeptides, dans le cas donné remplacés, sous l'action de l'agent condensant dans un milieu de dissolvant organique et présence de base à température de chambre; et les dérivés crus de la galanthamine obtenues se soumettent à précristallisation.

5. Méthode d'obtention des dérivés de la galanthamine suivant la revendication 4, se caractérisant avec le fait que, l'interaction de la galanthamine et de Boc-Gly-OCH₂CN s'effectue en la présence de DBU dans un milieu de dissolvant organique sélectionné du groupe CH₂Cl₂, DMF, CHCl₃, EtOAc, dimethylesulphoxyde.

6. Méthode d'obtention des dérivés de la galanthamine suivant la revendication 4, se caractérisant avec le fait que, l'ester 6-O-(Boc- glicyle)- galanthamine s'isole comme un produit chromatographique pur en commençant par aciduler avec un HCl dilué et ensuite par extraction des produits qui n'ont pas réagi avec un dissolvant organique sélectionné du groupe CH₂Cl₂, CHCl₃, EtOAc, alcalisation de la phase hydro-acide jusque pH=8 et suivi d'extraction du produit en objective avec un dissolvent organique, sélectionné du groupe CH₂Cl₂, CHCl₃, EtOAc.

7. Méthode d'obtention des dérivés de la galanthamine suivant la revendication 4, se caractérisant avec le fait que, le déblocage du Boc-groupe de protection s'effectue avec un acide, sélectionné du groupe TFA, acide chlorhydrique/dissolvent organique, HBr/AcOH et traitement suivant avec éther diethyle.

8. Méthode d'obtention des dérivés de la galanthamine suivant la revendication 4, se caractérisant avec le fait que, la condensation de l'ester 6-O-(Boc- glicyle)-galanthamine avec différents acides libres dipeptides, dans le cas donné remplacés, s'effectue sous l'action de l'agent condensant sélectionné du groupe HBTU ou TBTU ou TCTU dans un milieu de dissolvent organique sélectionné du groupe CH₂Cl₂, DMF, CHCl₃, EtOAc, dimethylesulphoxyde, et en présence de base sélectionnée du groupe de Et₃N, Me₃N, pyridine, tributalamine, DIPEA à température de chambre.

9. Utilisation des dérivés de la galanthamine suivant la revendication 1 pour l'obtention de produit pour inhibition de l'acétylcholinestérase, la butyle cholinestérase et la gamma-secretase.

10. Utilisation des composés avec la formule I, suivant la revendication 1 pour l'obtention du produit pour la prophylaxie et/ou traitement des maladies neuro-dégénératives.

11. Utilisation des composés avec la formule I, suivant la revendication 10 pour l'obtention du produit pour la prophylaxie et/ou traitement de la démence sénile et de la maladie de Alzheimer.

12. La composition pharmaceutique, contenant une quantité active thérapeutique d'au moins un des composés suivant la revendication 1 et un porteur acceptable du point de vu pharmaceutique et/ou des substances auxiliaires.
